# FASCICULE DE BREVET EUROPEEN

(11) **EP 0 487 878 B1**
(45) Date de publication et mention de la délivrance du brevet: **13.03.1996**
(21) Numéro de dépôt: 91117479.5
(22) Date de dépôt: 14.10.1991
(51) Int. Cl.: C12N 1/18, A21D 8/04, C12R 1/865

(54) **Levure de boulangerie**
Backhefe
Baker's yeast

(30) Priorité: 09.11.1990 CH 3565/90
(43) Date de publication de la demande: 03.06.1992
(73) Titulaire: SOCIETE DES PRODUITS NESTLE S.A., CH-1800 Vevey (CH)
(72) Inventeur: Gysler, Christof, Ch-1807 Blonay (CH); Hottinger, Herbert, CH-1807 Blonay (CH); Niederberger, Peter, CH-1066 Epalinges (CH)

(56) Documents cités:
- EP-A- 0 229 976
- Patent Abstracts of Japan, vol. 14, no. 304 (C-734)(4247), 29 June 1990
- Cereal Chemistry, vol. 64, no. 4, Aug. 1987, Minneapolis, US, pp. 269-275; A. Hino et al.

## Description

La présente invention a pour objet un procédé de construction d'une souche de levure de boulangerie présentant une propriété lti ainsi qu'une souche de levure construite par le présent procédé.

On connaît divers procédés de constructipn de souches de levures de boulangerie basés notamment sur la génétique traditionnelle et visant à conférer à ces souches des propriétés particulières utiles en boulangerie.

US 4547374, par exemple, décrit la construction, par hybridations sélectives, d'une souche d'espèce de Saccharomyces résistante à la congélation et utilisable comme levure de boulangerie dans la préparation d'une pâte à pain destinée à être congelée avant fermentation et cuisson.

US 4341871 décrit des hybrides de levure de boulangerie qui peuvent être déshydratés, même sous forme pressée, sans perte excessive de leur activité.

US 4643901 décrit des souches pures de levure de boulangerie capables de fermenter et faire lever des pâtes aussi bien sucrées que non sucrées et obtenues par hybridation par fusion protoplasmique de mutants petite.

On connaît également des articles de boulangerie du commerce destinés à être conservés au réfrigérateur avant fermentation et cuisson. Ces articles, tels que petits pains et croissants par exemple, comprennent cependant un agent de levage chimique.

La présente invention a pour but de proposer un procédé de construction de souches de levure de boulangerie, ainsi que des souches ainsi construites, qui présentent une propriété lti, autrement dit une propriété d'être inactives mais de survivre sous réfrigération (lti est le sigle de l'expression anglaise "low temperature inactive"), et qui soient utilisables comme levure de boulangerie dans la préparation d'articles de boulangerie destinés à être cuits au four juste avant consommation, après conservation au réfrigérateur, en chambre froide ou sur un rayon réfrigéré, par exemple.

A cet effet, dans une première forme de réalisation du procédé de construction d'une souche de levure de boulangerie présentant une propriété lti selon la présente invention, on soumet à un traitement mutagène une souche haploïde de Saccharomyces cerevisiae, on sélectionne au moins un mutant présentant une propriété lti, on réalise au moins un backcross avec une souche haploïde sauvage de Saccharomyces cerevisiae présentant un mating type opposé, on sélectionne au moins deux ségrégants de backcross présentant une propriété lti et des mating types opposés, on réalise au moins un croisement de tels ségrégants, et l'on sélectionne une souche diploïde ainsi obtenue présentant un potentiel de croissance, une propriété lti et une aptitude à lever une pâte.

Par l'expression "potentiel de croissance", on entend, dans le présent exposé, une aptitude à être cultivée avec un bon rendement et une bonne productivité par un processus de culture utilisable industriellement, notamment par le processus de culture traditionnel de la levure de boulangerie connu sous le nom de fed-batch (addition lente et progressive d'une solution de sucre à une suspension de levure sous aération, de manière à éviter la formation d'alcool au cours de la production de biomasse et à maximiser le rendement).

De même, par l'expression "une aptitude à lever une pâte", on entend une aptitude à transformer très lentement une pâte à température de réfrigération, par exemple par une production très lente de métabolites tels que du CO₂ qui peut être absorbé par la pâte et de l'alcool qui peut agir comme agent de conservation de la pâte, cette transformation très lente ayant pour conséquence que la pâte est susceptible de lever lorsqu'elle est mise directement au four après avoir été conservée au réfrigérateur, en chambre froide ou sur un rayon réfrigéré, par exemple.

Dans une deuxième forme de réalisation du procédé de construction d'une souche de levure de boulangerie présentant une propriété lti selon la présente invention, on soumet, le cas échéant, à un traitement mutagène une souche polyploïde de Saccharomyces cerevisiae, on fait sporuler cette souche, on sélectionne au moins un ségrégant présentant une propriété lti, on réalise au moins un backcross avec un autre ségrégant de cette souche présentant un mating type opposé, on sélectionne au moins deux ségrégants de backcross présentant une propriété lti et des mating types opposés, on réalise au moins un croisement de tels ségrégants, et l'on sélectionne une souche polyploïde ainsi obtenue présentant un potentiel de croissance, une propriété lti et une aptitude à lever une pâte.

On a en effet pu construire ainsi des souches de levure de boulangerie présentant la propriété d'être pratiquement inactives aux températures usuelles de réfrigération, notamment aux températures de environ 3 à 9 ou 10°C, de survivre cependant à ces températures et de retrouver ensuite leur activité à une température plus élevée, notamment dès environ 13-14°C, par exemple.

De telles souches de levure de boulangerie permettent donc de remplacer un agent de levage chimique dans la préparation d'articles de boulangerie destinés à être cuits au four juste avant consommation, après réfrigération. Elles permettent en particulier la préparation d'articles préformés tels que des petits pains, des croissants ou des fonds de pizza, ou d'une pâte à façonner par la ménagère, par exemple, qui présentent, après réfrigération et cuisson au four, des qualités organoleptiques comparables à celles présentées par de mêmes articles fraîchement levés sous l'action d'une levure de boulangerie traditionnelle du commerce et cuits au four.

De telles souches peuvent également être utilisées comme indicateur de dépassement de température dans des produits alimentaires à conserver sous réfrigération.

Pour mettre en oeuvre le présent procédé, on peut donc partir soit d'une souche haploïde de Saccharomyces cerevisiae telle que celles formant les levures de boulangerie traditionnelles de laboratoire, soit d'une souche polyploïde de Saccharomyces cerevisiae telle que celles formant les levures de boulangerie traditionnelles du commerce, par exemple.

Dans ladite première forme de réalisation du présent procédé où l'on part d'une souche haploïde de Saccharomyces cerevisiae, on soumet donc cette souche à un traitement mutagène. A cet effet, on peut faire croître des cellules de cette souche dans un milieu YPD contenant 2% de glucose, 1% d'extrait de levure et 2% de peptone, par exemple, et les cellules peuvent être traitées avec un agent mutagène tel que le méthanesulfonate d'éthyle (EMS) ou l'ICR-170, par exemple.

On sélectionne ensuite de préférence plusieurs mutants présentant une propriété lti, notamment une propriété d'inactivité mais de survivance à une température de environ 9 ou 10°C.

On réalise ensuite au moins un backcross du ou des mutants sélectionnés avec une souche haploïde sauvage de Saccharomyces cerevisiae présentant un mating type opposé, de manière à éviter des mutations indésirées que la souche haploïde de départ peut présenter au départ ou après ledit traitement mutagène, et/ou de manière à ne garder si possible une propriété lti due qu'à une seule mutation. Si l'on réalise plusieurs opérations de backcross, on peut sélectionner entre deux opérations successives au moins un ségrégant présentant une propriété lti, notamment une propriété d'inactivité mais de survivance à une température de environ 9 ou 10°C, et l'on peut soumettre ce ou ces ségrégants à la deuxième de ces deux opérations successives.

On sélectionne alors au moins deux ségrégants de backcross présentant des mating types opposés et une propriété lti, notamment une propriété d'inactivité mais de survivance à une température de environ 9 ou 10°C, et l'on réalise au moins un croisement de tels ségrégants.

On sélectionne enfin une souche diploïde de Saccharomyces cerevisiae ainsi obtenue présentant un potentiel de croissance, une propriété lti et une aptitude à lever une pâte. A cette étape finale, on peut en effet retenir des critères de sélection plus sévères et plus complets que ceux utilisés au cours des étapes précédentes. On peut en particulier soumettre ladite ou lesdites souches diploïdes à un test de croissance dans le processus de culture traditionnel ae la levure de boulangerie connu sous le nom de fed-batch.

On peut ensuite vérifier la propriété lti en soumettant ladite ou lesdites souches diploïdes à un test de production de CO₂ en milieu nutritif maltosé, autrement dit en milieu nutritif contenant du maltose comme source de carbone, en fonction du temps, par exemple tous les jours entre 3 et 7 d, et en fonction de la température, par exemple tous les degrés entre 3 et 14°C.

On peut enfin vérifier l'aptitude de la souche à lever une pâte en l'incorporant comme seul agent de levage dans une pâte à pizza, par exemple, en formant des fonds de pizza avec cette pâte, en les conservant quelques jours, voire quelques semaines, à température de réfrigération, puis en les cuisant au four. On peut également compléter ce test en vérifiant la production de CO₂ de la souche en milieu maltosé à une température de environ 20-30°C, par exemple.

En mettant en oeuvre cette première forme de réalisation du procédé selon la présente invention, on peut donc construire des souches qui présentent une propriété lti remarquable grâce à laquelle elles peuvent être utilisées pour confectionner des articles de boulangerie destinés à être cuits au four juste avant consommation, après conservation sous réfrigération. On peut construire en particulier des souches de levure de boulangerie Saccharomyces cerevisiae qui présentent un potentiel de croissance en processus fed-batch, une aptitude à lever une pâte, un niveau de production de CO₂ inférieur à 20 ml par g de levure pressée après 7 d en milieu maltosé réfrigéré à 3-10°C, et un niveau de production de CO₂ d'au moins 40 ml par g de levure pressée après 6 d en milieu maltosé tempéré à au moins 14°C.

Parmi diverses souches de Saccharomyces cerevisiae ainsi obtenues, on en a déposé trois à titre d'exemple, selon le Traité de Budapest, le 06.11.90, à la National Collection of Industrial and Marine Bacteria Ltd. (NCIMB), P.O.Box 31, 135 Abbey Road, ABERDEEN AB9 8DG, Ecosse (Royaume Uni) où elles ont reçu les Nos NCIMB 40328, 40329 et 40330.

Dans ladite deuxième forme de réalisation du présent procédé où l'on part d'une souche de Saccharomyces cerevisiae polyploïde, on soumet donc cette souche, le cas échéant, à un traitement mutagène. On a constaté en effet qu'il n'était pas toujours nécessaire de soumettre une telle souche à un traitement mutagène, car certaines levures de boulangerie du commerce, par exemple, peuvent présenter au départ des mutations susceptibles d'être mises en évidence au cours de la mise en oeuvre du présent procédé.

Au cas où l'on soumet une souche polyploïde à un traitement mutagène on peut, à cet effet, faire croître des cellules de cette souche dans un milieu YPD contenant 2% de glucose, 1% d'extrait de levure et 2% de peptone, par exemple, et les cellules obtenues peuvent être traitées avec un agent mutagène tel que le méthanesulfonate d'éthyle (EMS) ou l'ICR-70, par exemple.

Dans cette deuxième forme de réalisation du présent procédé, on fait donc sporuler une souche polyploïde de Saccharomyces cerevisiae après l'avoir, le cas échéant, soumise à un traitement mutagène. Pour faire sporuler cette souche on peut faire croître des cellules de cette souche durant un ou deux jours sur un milieu dit de présporulation tel qu'un milieu PSA contenant 0,8% d'extrait de levure, 0,3% de peptone, 10% de glucose et 2% d'agar. On peut ensuite les transférer et les maintenir durant 3-5 d sur un milieu de sporulation tel qu'un milieu SA contenant 1% d'acétate de potassium, 0,1% d'extrait de levure, 0,05% de glucose et 2% d'agar. On peut ensuite isoler des spores, par micromanipulation par exemple, et en obtenir des souches à ploïdie réduite, autrement dit des ségrégants, en les faisant germer sur un milieu YPD par exemple.

On sélectionne ensuite de préférence plusieurs ségrégants de ladite souche polyploïde présentant une propriété lti, notamment une propriété d'inactivité mais de survivance à une température de environ 9 - 10°C.

On réalise alors au moins un backcross du ou des ségrégants sélectionnés avec un autre ségrégant de ladite souche polyploïde ne présentant pas de propriété lti mais un mating type opposé, de manière à éviter des mutations indésirées que ladite souche polyploïde peut présenter au départ ou après l'éventuel traitement mutagène, et/ou de manière à ne garder si possible une propriété lti due qu'à une seule mutation. Si l'on réalise plusieurs opérations de backcross, on peut sélectionner entre deux opérations successives au moins un ségrégant présentant une propriété lti, notamment une propriété d'inactivité mais de survivance à une température de environ 9 - 10°C, et l'on peut soumettre ce ou ces ségrégants à la deuxième de ces deux opérations successives.

On sélectionne alors au moins deux ségrégants de backcross présentant des mating types opposés et une propriété lti, notamment une propriété d'inactivité mais de survivance à une température de environ 9 à 10°C, et l'on réalise au moins un croisement de tels ségrégants.

On sélectionne enfin une souche polyploïde de Saccharomyces cerevisiae ainsi obtenue présentant un potentiel de croissance, une propriété lti et une aptitude à lever une pâte. Pour ce faire, on peut retenir les mêmes critères de sélection et soumettre ladite ou lesdites souches polyploïdes aux mêmes tests que ceux décrits ci-dessus à l'étape finale de ladite première forme de réalisation du présent procédé.

En mettant en oeuvre cette deuxième forme de réalisation du procédé selon la présente invention, on peut donc construire des souches qui présentent une propriété lti remarquable grâce à laquelle elles peuvent être utilisées pour confectionner des articles de boulangerie destinés à être cuits au four juste avant consommation, après conservation sous réfrigération. On peut construire en particulier des souches de levure de boulangerie Saccharomyces cerevisiae qui présentent un potentiel de croissance en processus fed-batch, une aptitude à lever une pâte, un niveau de production de CO₂ inférieur à 30 ml par g de levure pressée après 7 d en milieu maltosé réfrigéré à 3-9°C, et un niveau de production de CO₂ d'au moins 60 ml par g de levure pressée après 6 d en milieu maltosé tempéré à au moins 13°C.

Parmi diverses souches de Saccharomyces cerevisiae ainsi obtenues, on en a déposé deux à titre d'exemple, selon le Traité de Budapest, le 06.11.90, à la National Collection of Industrial and Marine Bacteria Ltd. (NCIMB), P.O.Box 31, 135 Abbey Road, ABERDEEN AG9 8DG, Ecosse (Royaume Uni) où elles ont reçu les Nos NCIMB 40331 et 40332.

Les exemples ci-après sont présentés à titre d'illustration du procédé et des souches obtenues par le procédé selon la présente invention. Les pourcentages et parties y sont donnés en poids sauf indication contraire.

Ces exemples sont précédés d'une description de divers tests, de la composition de divers milieux utilisés, d'une brève description des différentes figures du dessin annexé, et d'un exemple comparatif.

### TESTS

### 1. Test de croissance

Pour simuler un essai réel de croissance dans le processus de culture traditionnel de la levure de boulangerie connu sous le nom de fed-batch, on a conçu un test de croissance sur divers milieux de culture contenant des sources de carbone non fermentables telles que l'acide lactique (milieu S-lac), l'éthanol (milieux S-EtOH 2% et S-EtOH 1%), et le glycérol (milieu YPG).

Le raisonnement à la base de ce test est que la levure de boulangerie accumule des métabolites carbonés non fermentables en réponse à une vitesse d'addition critique d'une solution d'un sucre fermentable tel que le saccharose, au cours du processus fed-batch. Ces métabolites ont un effet inhibiteur et toxique sur le métabolisme et sur la respiration de la levure. Des souches présentant un potentiel de croissance élevé peuvent accumuler ces métabolites carbonés toxiques à des vitesses d'addition plus grandes et elles sont moins sensibles à une accumulation de ces métabolites que des souches présentant un potentiel de croissance moins élevé.

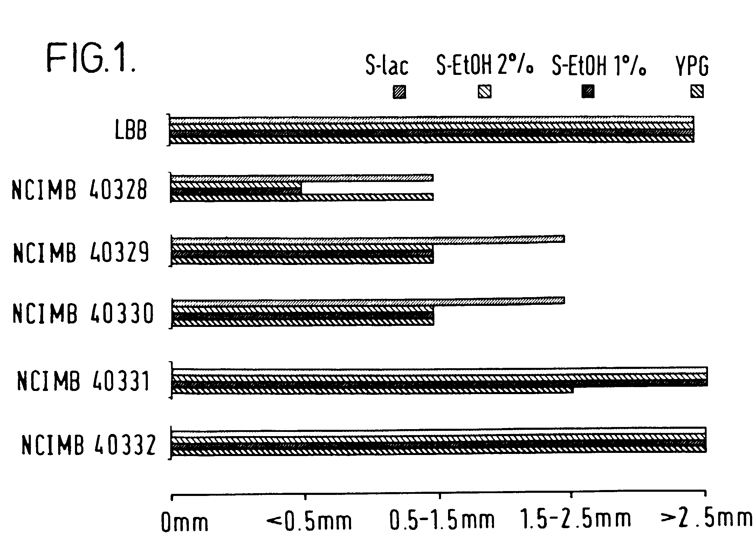

Les résultats de ce test de croissance sont présentés sur la fig. 1 en termes de dimensions que présentent les colonies après incubation de cellules d'une souche sur des plaques desdits milieux durant 3 d à 30°C. Les bandes horizontales correspondant à chaque milieu s'arrêtent à des marques 0; 0,5 mm; 0,5-1,5 mm; 1,5-2,5 mm; 2,5 mm indiquant une dimension moyenne observée des colonies.

### 2. Test de production de CO₂

On réalise ce test dans un appareil spécialement conçu pour cela qui comprend un bloc à gradient de température présentant des loges à températures variables, par exemple, dans lesquelles on peut introduire l'extrémité inférieure de tubes de fermentation. Ces tubes présentent une extrémité supérieure fermée et graduée, ainsi qu'un ballon d'expansion branché sur le côté. Le CO₂ produit par la levure s'accumule dans l'extrémité supérieure graduée de chaque tube, le milieu de culture déplacé par l'accumulation du gaz pouvant passer dans le ballon d'expansion.

Pour réaliser ce test, on inocule 2 ml d'une culture d'une nuit sur milieu YPD de la souche à tester dans 200 ml d'un premier milieu contenant 0,67% d'une base azotée sans acides aminés, telle que le produit commercialiés par la maison Difco sous la désignation "yeast nitrogen base w/o amino acids", par exemple, 0,5% d'extrait de levure, 2% de saccharose, 1% de succinate de sodium et de l'acide chlorhydrique concentré pour ajouter le pH à 4,5, dans un flacon de 500 ml. On incube sous agitation durant 24 h à 30°C.

On sépare les cellules par centrifugation sous 6000 g à 20°C durant 5 min et on les met en suspension dans 200 ml d'un deuxième milieu contenant 0,67% d'une base azotée sans acides aminés, 0,3% d'extrait de levure et 0,3% de saccharose, 1% de succinate de sodium et de l'acide chlorhydrique concentré pour ajuster le pH à 4,5, dans un flacon de 500 ml. On incube sous agitation durant 24 h à 30°C.

On sépare les cellules par centrifugation sous 6000 g à 4°C durant 5 min et l'on lave deux fois le culot de cellules de levure obtenu avec 50 ml d'eau distillée.

On suspend les cellules dans 10 ml d'eau distillée et on les transfère dans des tubes de polypropylène de 15 ml gradués et prépesés. On les centrifuge sous 3000 g à 4°C durant 10 min. On égoutte les tubes, on pèse les culots de levure et on les met en suspension, à raison de 0,61 g de culot de levure équivalant à 0,5 g de levure pressée présentant une teneur en matière sèche de environ 27%, par ml, dans un troisième milieu contenant 0,67% de base azotée sans acides aminés, 2% de maltose, 1% de succinate de sodium et de l'acide chlorhydrique concentré pour ajuster le pH à 4,5. On introduit 0,5 ml (pour des températures ≥ 10°C) ou 1 ml (pour des températures < 10°C) dans des tubes de fermentation tels que décrits ci-dessus que l'on a remplis au préalable chacun avec 50 ml dudit troisième milieu, autrement dit du milieu maltosé, et que l'on a refroidis à 4°C.

On incube les tubes de fermentation aux températures désirées dans le bloc à gradient de température décrit ci-dessus. On note la production de CO₂ à intervalles choisis après avoir plongé les tubes quelques secondes dans un bain à agitation sonique pour dégager des bulles de CO₂ retenues dans le milieu liquide.

### 3. Test d'aptitude à lever une pâte

On prépare une pâte en mélangeant 30 parties d'eau, 60 parties de farine blanche de blé tendre, 1,4 partie de chlorure de sodium et 7,6 parties d'huile d'arachide. On y incorpore la souche à tester, à raison de 1 partie de levure pressée. On en forme des fonds de pizza de 20 cm de diamètre et 0,5 cm d'épaisseur. On les conserve 21 d à 8°C en emballage plastique scellé.

On sort alors les fonds de pizza de leur emballage et on les cuit durant 15 min au four à 180°C.

Le test est considéré comme réussi si les fonds de pizza présentent alors une épaisseur de environ 2 cm et des qualités organoleptiques, notamment un goût et une texture comparables à celles présentées par des fonds de pizzas analogues préparés avec une pâte fraîchement levée sous l'action d'une levure de boulangerie traditionnelle du commerce.

### MILIEUX

### YPD

glucose 2%
extrait de levure 1%
peptone 2%

### PSA

glucose 10%
extrait de levure 0,8%
peptone 0,3%
agar 2%

### SA

glucose 0,05%
extrait de levure 0,1%
acétate de potassium 1%
agar 2%

### S-lac

0,67% base azotée sans acides aminés
0,5% acide lactique
2% agar

### S-EtOH 1% ou 2%

0,67% base azotée sans acides aminés
1% ou 2% ethanol
2% agar

### YPG

glycerol 2%
extrait de levure 1%
peptone 2%

### DESSINS

### Fig. 1:

Représentation linéaire de la dimension de colonies obtenues par croissance sur milieux S-lac, S-EtOH 1% et 2%, et YPG de cellules des souches NCIMB 40328, 40329, 40330, 40331, 40332 et "levure boulangère bleue" (LBB, pour comparaison).

### Fig. 2 à Fig.7:

Représentation tridimensionnelle du niveau de production de CO₂ en milieu maltosé en fonction de la température et de la durée pour les souches NCIMB 40328 (Fig.2), NCIMB 40329 (Fig.3), NCIMB 40330 (Fig.4), NCIMB 40331 (Fig.5), NCIMB 40332 (Fig.6), et "levure boulangère bleue" (LBB, pour comparaison, Fig.7)

### Fig. 8:

Représentation bidimensionnelle de la production de CO₂ en milieu maltosé à 30°C en fonction du temps pour les souches NCIMB 40328, 40329, 40330, 40332 et levure boulangère bleue (LBB, pour comparaison)

### Exemple comparatif

A titre de comparaison, on soumet une souche de levure de boulangerie du commerce, en l'occurence une souche formant la levure de boulangerie commercialisée par la maison Fould Springer sous la dénomination "levure boulangère bleue" (LBB) aux mêmes tests que ceux utilisés plus haut pour sélectionner lesdites souches diploïdes et polyploïdes présentant un potentiel de croissance, une propriété lti et une aptitude à lever une pâte, autrement dit les tests 1 à 3 décrits plus haut.

Les résultats du test comparatif de croissance présentés sur la Fig. 1 montrent de manière attendue que la souche LBB a un très bon potentiel de croissance dans le processus de culture traditionnel de la levure de boulangerie dit fed-batch.

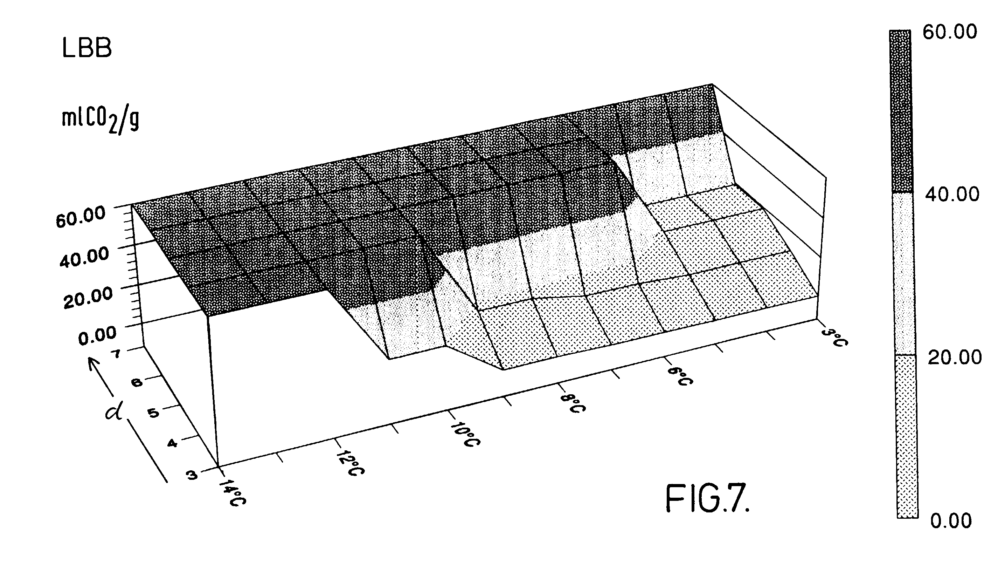

Les résultats du test comparatif de production de CO₂ sont présentés à la Fig. 7 où l'on voit que la production de CO₂ en milieu maltosé dépasse 40 ml par g de levure pressée dès 6-7 d à 3-5°C, dès 5-6 d à 5-9°C et dès 3-4 d à 10°C.

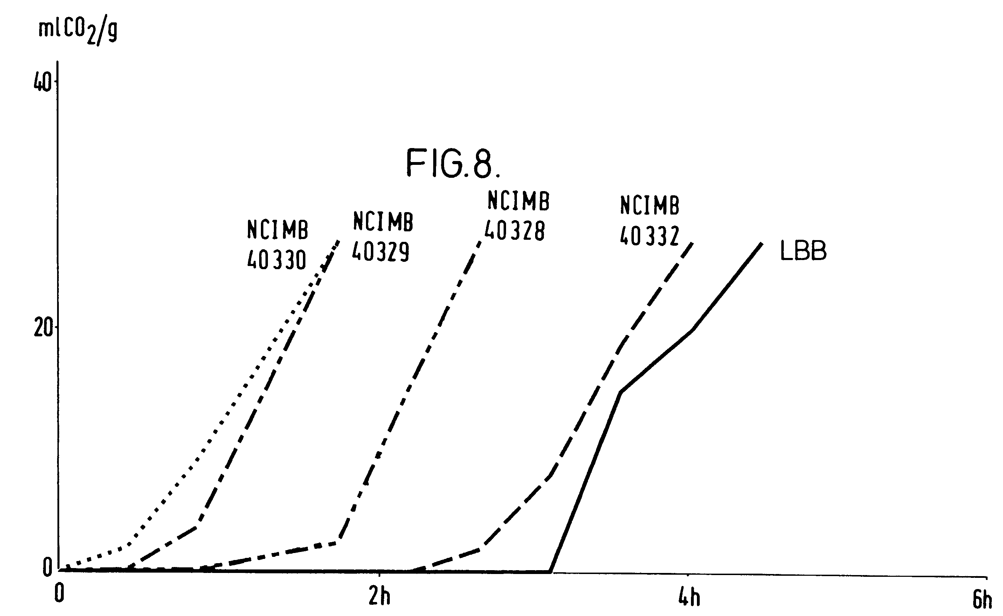

On voit en outre sur la fig. 8 que la production de CO₂ en milieu maltosé de la souche LBB dépasse rapidement 20 ml par g de levure pressée dès environ 4 h à 30°C.

Les résultats du test comparatif d'aptitude à lever une pâte montrent que la souche LBB ne se prête pas à une conservation sous réfrigération avant cuisson au four. L'emballage en plastique scellé dans lequel est emballé un fond de pizza obtenu en utilisant la souche LBB se gonfle comme un ballon dès 1 d à 8°C.

Par contre, on constate qu'aucune différence de qualité n'est pratiquement perceptible entre les fonds de pizza obtenus en utilisant des souches construites par le présent procédé et soumis au test ci-dessus d'aptitude à lever une pâte, d'une part, et les fonds de pizza obtenus en utilisant la souche LBB et soumis au même test dont on supprime l'étape de conservation sous réfrigération.

### Exemple 1

On part d'une souche haploïde de Saccharomyces cerevisiae telle que celles formant les levures de boulangerie traditionnelles de laboratoire, notamment d'une souche présentant le génotype MATa arg4-17 his4-38 lys1-1 met8-1 trp1-1 mal, que l'on a déposée selon le Traité de Budapest le 06.11.90 à la National Collection of Industrial and Marine Bacteria Ltd. (NCIMB), P.O.Box 31, 135 Abbey Road, ABERDEEN AB9 8DG, Ecosse (Royaume Uni) où elle a reçu le No NCIMB 40333.

On soumet cette souche à un traitement mutagène à l'EMS. Pour ce faire, on fait croître des cellules de cette souche dans 5 ml de milieu de culture YPD jusqu'à la phase stationnaire, on les lave une fois avec un tampon phosphate de potassium 100 mM à pH 7,0 et on les met en suspension dans le même tampon à raison de 10⁸ cellules/ml.

On ajoute 3% en volume d'EMS à la suspension et on la laisse réagir durant 1 h à 30°C sous vigoureuse agitation. On arrête le traitement par dilution de la suspension dans 10 fois son volume d'une solution à 5% de thio-sulfate de sodium. On répand alors les cellules sur milieu YPD solide et on les y cultive durant 2 d à 30°C. On les repique ensuite sur milieu YPD solide et on les y cultive durant 3 semaines à 10°C pour tester leur propriété lti.

On sélectionne alors quelques mutants stables présentant une propriété d'inactivité mais de survivance à cette température.

On réalise un backcross d'un de ces mutants présentant un mating type MATa avec une souche haploïde sauvage de Saccharomyces cerevisae telle que, par exemple, la souche GRF18 présentant le génotype MATα can1 his3-11,15 leu2-3,112, qui est bien connue de l'homme du métier (cf. G.R.Fink, Whitehead Institute, Nine Cambridge Center, Cambridge, Massachusetts 02 142, USA).

On sélectionne un ségrégant de ce backcross présentant une propriété lti fortement marquée à 10°C ainsi que le génotype MATa lys1 his3/4 trp1 mal.

On réalise un backcross de ce ségrégant avec la même souche sauvage que celle utilisée pour le backcross d'un mutant de la souche de départ.

On sélectionne alors deux ségrégants de ce backcross présentant une propriété lti marquée à 10°C ainsi que chacun le génotype MATα leu2 his3/4 mal.

On réalise un backcross de chacun de ces deux ségrégants avec une souche haploïde sauvage de Saccharomyces cerevisiae présentant au moins un gène MAL, telle que la souche 1403-7A, par exemple, qui est bien connue de l'homme du métier (cf Yeast Genetics Stock Center, 6ème édition du catalogue, par Robert Mortimer, Department of Biophysics and Medical Physics, University of California, Berkeley, CA 94720, USA).

On sélectionne alors un ségrégant de chacun de ces backcross, présentant chacun une propriété lti fortement marquée à 10°C ainsi que l'un un génotype MATa MAL et l'autre un génotype MATα leu2 MAL.

On réalise un croisement de ces deux ségrégants et l'on sélectionne diverses souches diploïdes de Saccharomyces cerevisiae issues de ce croisement qui présentent un potentiel de croissance, une propriété lti et une aptitude à lever une pâte.

Parmi diverses souches ainsi obtenues, on a déposé, à titre d'exemple, la souche NCIMB 40328 mentionnée plus haut. Cette souche présente un potentiel de croissance relativement modeste dans le processus de culture traditionnel de la levure de boulangerie dit fed-batch, comme on peut le voir à la fig. 1 qui illustre les résultats obtenus selon le test de croissance ci-dessus.

Elle présente cependant une bonne aptitude à fermenter une pâte, car elle réussit bien le test correspondant ci-dessus.

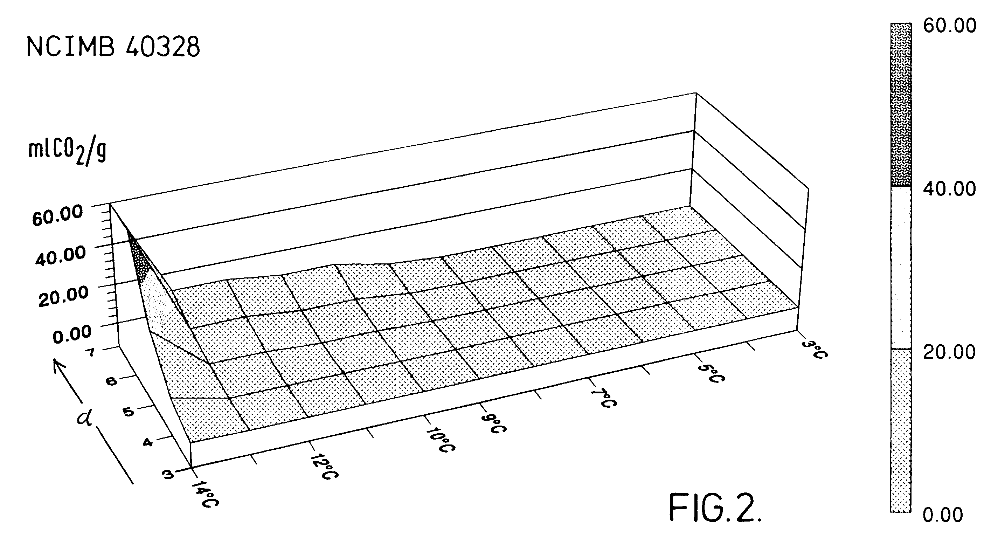

Elle présente enfin une propriété lti marquée, comme on peut le voir à la fig. 2 qui représente donc sous forme de diagramme tridimensionnel son niveau de production de CO₂ en fonction de la température et de la durée de fermentation en milieu maltosé, tel que déterminé par le test de production de CO₂ décrit en détail ci-dessus. On voit en effet qu'elle est pratiquement inactive entre 3 et 10°C durant au moins environ une semaine, mais qu'elle survit et qu'elle est en mesure de reprendre une activité notable dès environ 13-14°C après environ 6 à 7 d. On voit en particulier que son niveau de production de CO₂ est encore voisin de zéro après 7 d entre 3 et 8°C, et qu'il ne monte qu'à environ 8 ml par g de levure pressée après 7 d à 10°C. Par contre, son niveau de production de CO₂ en milieu maltosé monte à plus de 40 ml/g dès 13-14°C après 6 d.

On constate également, comme on peut le voir à la fig.8, que son niveau de production de CO₂ en milieu maltosé monte rapidement à plus de 20 ml/g dès environ 2 h à 30°C.

Cette souche NCIMB 40328 présente en outre les caractéristiques suivantes:

### Morphologie:

Cellules elliptiques. Certaines cellules s'agrandissent et forment un pseudomycelium.

### Fermentation des sucres:

La souche est capable de fermenter le saccharose, le maltose et le glucose.

### Exemple 2:

On part de la même souche de départ et l'on procède de la même manière qu'à l'exemple 1 jusqu'à ladite sélection de deux ségrégants de backcross présentant une propriété lti marquée à 10°C ainsi que le génotype MATα leu2 his3/4 mal.

On réalise un backcross de l'un de ces deux ségrégants avec une souche haploïde sauvage de Saccharomyces cerevisiae telle que, par exemple, la souche X2180-1A présentant le génotype MATa CUP1 SUC2 gal2 mal mel, qui est bien connue de l'homme du métier (cf. Yeast Genetics Stock Center, 6ème édition du catalogue).

On sélectionne un ségrégant de ce backcross présentant une propriété lti marquée à 10°C ainsi que le génotype MATα his3/4 leu2 mal.

On réalise un backcross de ce ségrégant avec la même souche X2180-1A que ci-dessus pour éliminer les mutations auxotrophiques his3/4 et leu2.

On sélectionne un ségrégant de ce backcross présentant le génotype MATα mal et une propriété lti notable à 10°C due à une mutation unique, autrement dit présentant une ségrégation 2:2 parfaite en ce qui concerne cette propriété lti.

On réalise un backcross de ce ségrégant avec une souche haploïde sauvage de Saccharomyces cerevisiae présentant au moins un gène MAL, telle que la souche 1403-7A, par exemple, qui est bien connue de l'homme du métier (cf Yeast Genetics Stock Center, 6ème édition du catalogue).

On sélectionne d'une part un ségrégant de ce backcross présentant une propriété lti marquée à 10°C ainsi que le génotype MATα MAL, et d'autre part deux ségrégants de ce backcross présentant des propriétés lti respectives marquées et fortement marquées à 10°C ainsi que chacun le phénotype MATa ura3 mal.

On réalise un croisement de chacun de ces deux derniers ségrégants avec le premier et l'on sélectionne diverses souches diploïdes de Saccharomyces cerevisiae issues de ces deux croisements qui présentent un potentiel de croissance, une propriété lti et une aptitude à lever une pâte.

Parmi diverses souches ainsi obtenues, on a déposé, à titre d'exemple, les souches NCIMB 40329 et NCIMB 40330 mentionnées plus haut et issues chacune d'un de ces deux croisements. Ces deux souches présentent un potentiel de croissance relativement bon dans le processus de culture traditionnel de la levure de boulangerie dit fed-batch, comme on peut le voir à la fig. 1.

Elles présentent également une bonne aptitude à lever une pâte, car elles réussissent bien le test correspondant ci-dessus.

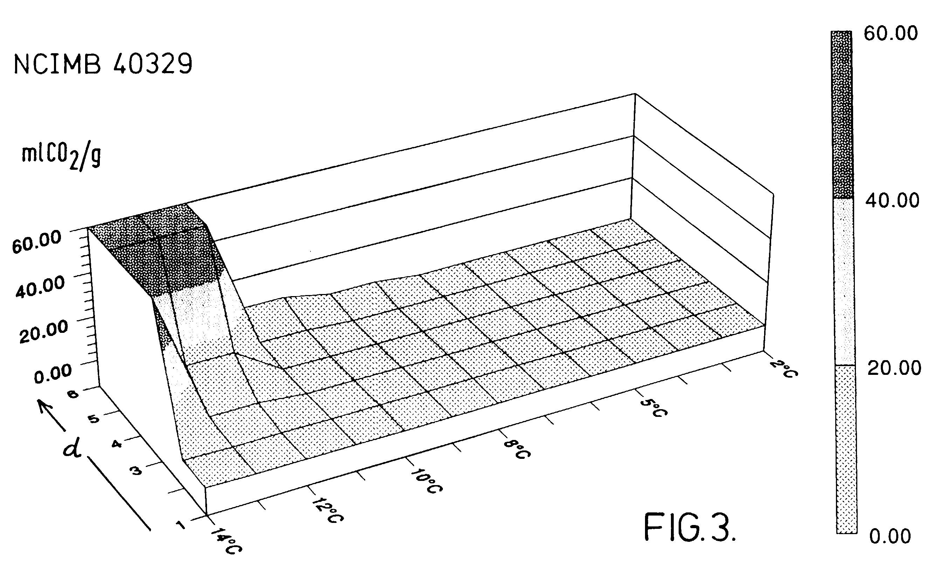

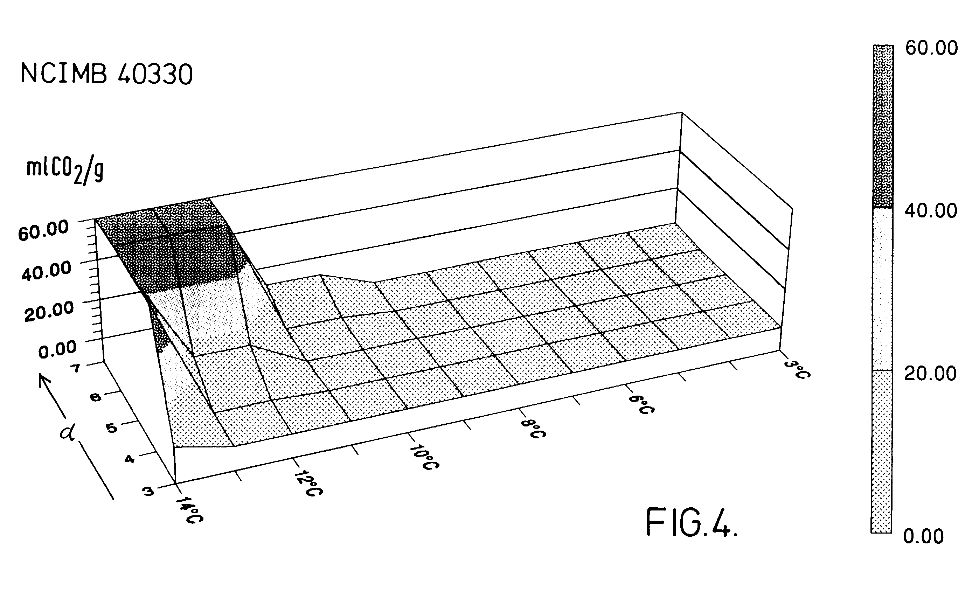

Elles présentent enfin une propriété lti marquée. On voit en effet aux fig. 3 et 4 qu'elles sont pratiquement inactives en milieu maltosé entre 3 et 10°C durant au moins environ une semaine, mais qu'elles survivent et qu'elles sont en mesure de reprendre une activité notable dès environ 11-14°C après environ 5 à 7 d. On voit en particulier que leur niveau de production de CO₂ est encore voisin de zéro après 7 d entre 3 et 9°C, et qu'il ne monte qu'à environ 12 ml par g de levure pressée après 7 d à 10°C. Par contre, leur niveau de production de CO₂ monte à plus de 40 ml/g dès 12-14°C après 6 d.

On constate également, comme on peut le voir à la fig.8, que leur niveau de production de CO₂ en milieu maltosé monte rapidement à plus de 20 ml/g dès environ 1,5 h à 30°C.

Ces souches présentent en outre les caractéristiques suivantes:

### NCIMB 40329

### Morphologie:

Cellules elliptiques. Taille des cellules relativement homgène.

### Fermentation des sucres:

Capable de fermenter le saccharose, le maltose et le glucose.

### NCIMB 40330

### Morphologie:

Cellules elliptiques. Taille des cellules relativement homogène.

### Fermentation des sucres:

Capable de fermenter le saccharose, le maltose et le glucose.

### Exemple 3

On part d'une souche polyploïde de Saccharomyces cerevisiae du commerce telle que celle formant la levure de boulangerie commercialisée par la maison Fould Springer sous la dénomination "levure boulangère bleue", dont les cellules contiennent trois à quatre fois plus de DNA que les cellules d'une souche haploïde de Saccharomyces cerevisiae.

Pour faire sporuler cette souche, on fait croître de ses cellules sur un milieu de présporulation PSA durant deux d à 30°C. On les transfère ensuite sur un milieu de sporulation SA où on les maintient durant 4 d à 30°C.

On isole alors des spores et on les fait germer sur un milieu de culture YPD pour obtenir des souches, autrement dit des ségrégants, à ploïdie réduite.

On sélectionne un de ces ségrégants présentant une propriété lti fortement marquée à 10°C et présentant par ailleurs le mating type MATα .

On réalise un backcross de ce ségrégant avec un autre desdits ségrégants à ploïdie réduite qui ne présente pas une propriété lti mais le mating type MATa.

On sélectionne plusieurs ségrégants de ce backcross qui présentent une propriété lti fortement marquée à 10°C et un mating type.

On réalise plusieurs croisements entre ségrégants présentant des mating types opposés et l'on sélectionne diverses souches polyploïdes de Saccharomyces cerevisiae issues de ces croisements qui présentent un potentiel de croissance, une propriété lti et une aptitude à lever une pâte.

Parmi diverses souches ainsi obtenues, on a déposé, à titre d'exemple, les souches NCIMB 40331 et NCIMB 40332 mentionnées plus haut. Ces deux souches présentent en particulier un très bon potentiel de croissance dans le processus de culture traditionnel de la levure de boulangerie dit fed-batch, comme on peut le voir à la fig. 1.

Elles présentent également une bonne aptitude à lever une pâte, car elles réussissent bien le test correspondant ci-dessus.

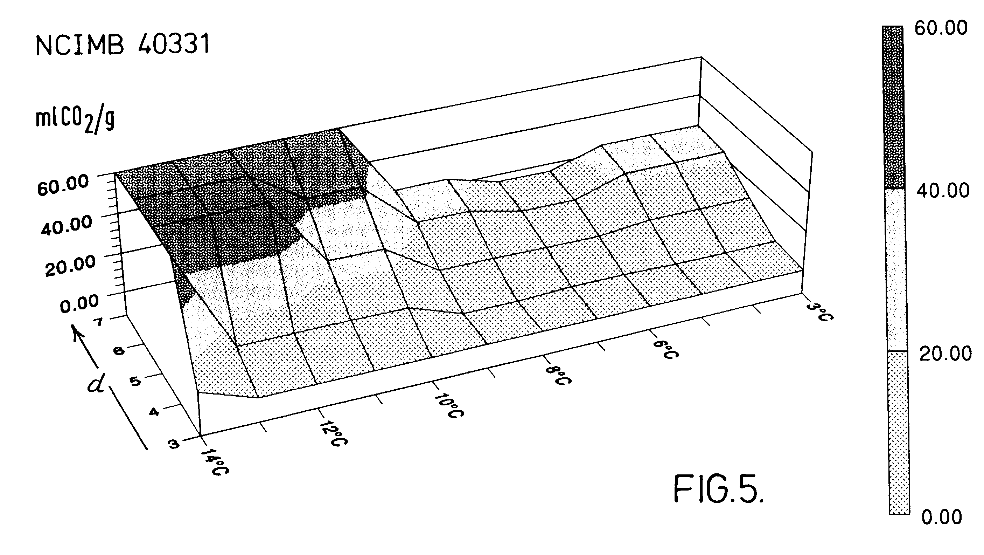

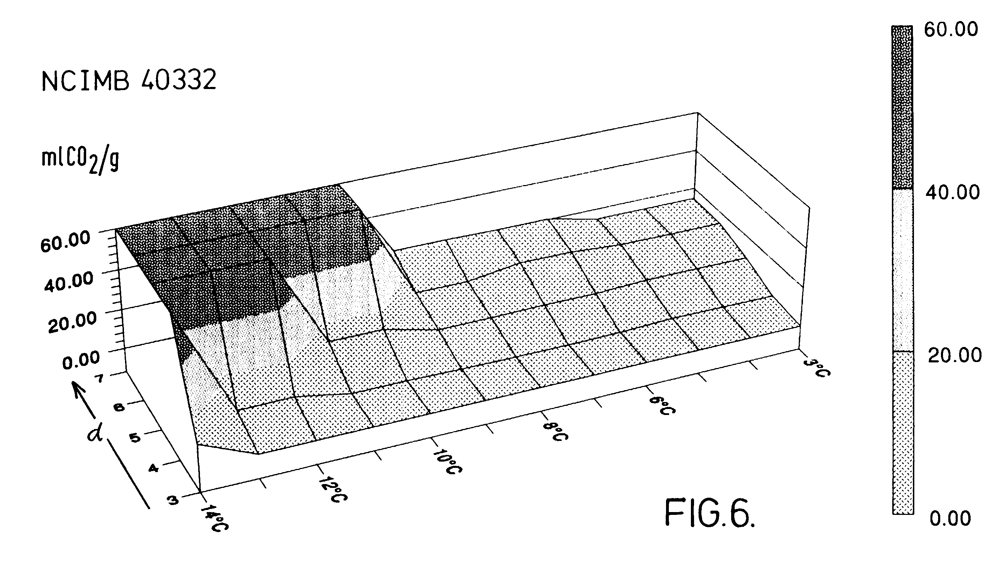

Elles présentent enfin une propriété lti marquée. On voit en effet aux fig. 5 et 6 qu'elles sont pratiquement inactives en milieu maltosé entre 3 et 9°C durant au moins 4 à 5 d, mais qu'elles survivent et qu'elles sont en mesure de reprendre une activité notable dès environ 10-13°C après environ 5-7 d. On voit en particulier que leur niveau de production de CO₂ est encore voisin de zéro après 5 d entre 3 et 9°C, et qu'il ne monte qu'à environ 20-25 ml par g de levure pressée après 7 d à 3-9°C. Par contre, leur niveau de production de CO₂ monte à plus de 60 ml/g dès 12-14°C après 5 d.

On constate également, comme on peut le voir à la fig. 8, que le niveau de production de CO₂ de la souche NCIMB 40332 en milieu maltosé monte rapidement à plus de 20 ml/g dès environ 4 h à 30°C.

Ces souches présentent en outre les caractéristiques suivantes:

### NCIMB 40331

### Morphologie:

Cellules rondes. Taille des cellules homogène.

### Fermentation des sucres:

Capable de fermenter le saccharose, le maltose et le glucose.

### NCIMB 40332

### Morphologie:

Cellules rondes. Taille des cellules homogène.

### Fermentation des sucres:

Capable de fermenter le saccharose, le maltose et le glucose.

## Revendications

1. Procédé de construction d'une souche de levure de boulangerie diploïde présentant une propriété lti, c'est à dire la propriété de produire moins de 20 ml de CO₂ par g de levure pressée après 7d en milieu maltosé réfrigéré à 3-10°C et de retrouver son activité dès 13-14°C, dans lequel on soumet à un traitement mutagène une souche haploïde de Saccharomyces cerevisiae, on sélectionne au moins un mutant présentant une propriété lti, on réalise au moins un backcross avec une souche haploïde sauvage de Saccharomyces cerevisiae présentant un mating type opposé, on sélectionne au moins deux ségrégants de backcross présentant une propriété lti et des mating types opposés, on réalise au moins un croisement de tels ségrégants, et l'on sélectionne une souche diploïde ainsi obtenue présentant un potentiel de croissance, une propriété lti et une aptitude à lever une pâte.

2. Procédé selon la revendication 1, caractérisé par le fait que pour vérifier la propriété lti de ladite souche diploïde à sélectionner, on la soumet à un test de production de CO₂ en milieu nutritif contenant du maltose comme source de carbone, durant plusieurs jours, à des températures comprises entre 3 et 14°C.

3. Procédé de construction d'une souche de levure de boulangerie polyploïde présentant une propriété lti, c'est à dire la propriété de produire moins de 30 ml de CO₂ par g de levure pressée après 7d en milieu maltosé réfrigéré à 3-9°C et de retrouver son activité dès 13-14°C, dans lequel on soumet, le cas échéant, à un traitement mutagène une source polyploïde de Saccharomyces cerevisiae, on fait sporuler cette souche, on sélectionne au moins un ségrégant présentant une propriété lti, on réalise au moins un backcross avec un autre ségrégant de cette souche présentant un mating type opposé, on sélectionne au moins deux ségrégants de backcross présentant une propriété lti et des mating types opposés, on réalise au moins un croisement de tels ségrégants, et l'on sélectionne une souche polyloïde ainsi obtenue présentant un potentiel de croissance, une propriété lti et une aptitude à lever une pâte.

4. Procédé selon la revendication 3, caractérisé par le fait que pour vérifier la propriété lti de ladite souche polyploide à sélectionner, on la soumet à un test de production de CO₂ en milieu nutritif contenant du maltose comme source de carbone, durant plusieurs jours, à des températures comprises entre 3 et 14°C.

5. Souche diploïde de levure de boulangerie Saccharomyces cerevisiae, qui présente un potentiel de croissance en processus fed-batch, une aptitude à lever une pâte, un niveau de production de CO₂ inférieur à 20 ml par g de levure pressée après 7 d en milieu maltosé réfrigéré à 3-10°C, et un niveau de production de CO₂ d'au moins 40 ml par g de levure pressée après 6 d en milieu maltosé tempéré à au moins 14°C.

6. Souche polyploïde de levure de boulangerie Saccharomyces cerevisiae, qui présente un potentiel de croissance en processus fed-batch, une aptitude à lever une pâte, un niveau de production de CO₂ inférieur à 30 ml par g de levure pressée après 7 d en milieu maltosé réfrigéré à 3-9°C, et un niveau de production de CO₂ d'au moins 60 ml par g de levure pressée après 6 d en milieu maltosé tempéré à au moins 13°C.

7. Souche de levure de boulangerie selon la revendication 5, choisie dans le groupe formé par les souches de Saccharomyces cerevisiae NCIMB 40328, 40329 et 40330.

8. Souche de levure de boulangerie selon la revendication 6, choisie dans le groupe formé par les souches de Saccharomyces cerevisiae NCIMB 40331 et 40332.

9. Utilisation d'une souche de levure de boulangerie selon l'une des revendications 5 à 8 dans la production d'articles de boulangerie à cuire au four après conservation sous réfrigération.

10. Utilisation d'une souche de levure de boulangerie selon l'une des revendications 5 à 8 comme indicateur de dépassement de température dans des produits alimentaires à conserver sous réfrigération.

## Patentansprüche

1. Verfahren zur Entwicklung eines diploiden Backhefe-Stamms, der LTI-Eigenschaft aufweist, d.h. die Eigenschaft, weniger als 20 ml CO₂ pro g Preßhefe nach 7 Tagen in einem auf 3 bis 10°C gekühlten Maltosemilieu zu entwickeln und seine Aktivität bei 13-14°C wiederzuerlangen, bei dem man einen haploiden Stamm von Saccharomyces cerevisiae einer mutagenen Behandlung unterwirft, wenigstens eine Mutante selektioniert, die LTI-Eigenschaft aufweist, wenigstens eine Rückkreuzung mit einem haploiden Wildstamm von Saccharomyces cerevisiae durchführt, der einen entgegengesetzten Paarungstyp repräsentiert, wenigstens zwei Segreganten der Rückkreuzung selektioniert, die LTI-Eigenschaft zeigen und vom entgegengesetzten Paarungstyp sind, wenigstens eine Kreuzung dieser Segreganten durchführt, und einen auf diese Weise erhaltenen Stamm selektioniert, der wachstumsfähig ist, LTI-Eigenschaft und eine Teigtreibfähigkeit aufweist.

2. Verfahren anch Anspruch 1, dadurch gekennzeichnet, daß man zur Feststellung der LTI-Eigenschaft des zu selektionierenden diploiden Stamms diesen während einiger Tage bei Temperaturen zwischen 3 und 14°C einem Test der CO₂-Erzeugung in einem Nährmedium unterwirft, das als Kohlenstoffquelle Maltose enthält.

3. Verfahren zur Entwicklung eines polyploiden Backhefe-Stamms, der LTI-Eigenschaft aufweist, d.h. die Eigenschaft, weniger als 30 ml CO₂ pro g Preßhefe nach 7 Tagen in einem auf 3 bis 9°C gekühlen Maltosemilieu zu entwickeln und seine Aktivität bei 13-14°C wiederzuerlangen, bei dem man, gegebenenfalls, einen polyploiden Stamm von Saccharomyces cerevisiae einer mutagenen Behandlung unterwirft, diesen Stamm sporulieren läßt, wenigstens einen Segreganten selektioniert, der LTI-Eigenschaft aufweist, wenigstens eine Rückkreuzung mit einem anderen Segreganten dieses Stamms durchführt, der einen entgegengesetzten Paarungstyp repräsentiert, wenigstens zwei Segreganten der Rückkreuzung selektioniert, die LTI-Eigenschaft zeigen und vom entgegengesetzten Paarungstyp sind, wenigstens eine Kreuzung dieser Segreganten durchführt, und einen auf diese Weise erhaltenen polyploiden Stamm selektioniert, der wachstumsfähig ist, LTI-Eigenschaft und eine Teigtreibfähigkeit aufweist.

4. Verfahren nach Anspruch 3, dadurch gekennzeichnet, daß man zur Feststellung der LTI-Eigenschaft des zu selektionierenden polyploiden Stamms diesen während einiger Tage bei Temperaturen zwischen 3 und 14°C einem Test der CO₂-Erzeugung in einem Nährmedium unterwirft, das als Kohlenstoffquelle Maltose enthält.

5. Diploider Stamm der Backhefe Saccharomyces cerevisiae, der beim Zulaufverfahren wachstumsfähig ist und eine Teigtreibfähigkeit, ein Produktionsniveau von CO₂ von weniger als 20 ml pro g Preßhefe nach 7 Tagen in einem auf 3-10°C gekühlten Maltosemilieu und ein Produktionsniveau von CO₂ von wenigstens 40 ml pro g Preßhefe nach 6 Tagen in einem Maltosemilieu einer Temperatur von wenigstens 14°C aufweist.

6. Polyploider Stamm der Backhefe Saccharomyces cerevisiae, der beim Zulaufverfahren wachstumsfähig ist und eine Teigtreibfähigkeit, ein Produktionsniveau von CO₂ von weniger als 30 ml pro g Preßhefe nach 7 Tagen in einem auf 3-9°C gekühlten Maltosemilieu und ein Produktionsniveau von CO₂ von wenigstens 60 ml pro g Preßhefe nach 6 Tagen in einem Maltosemilieu einer Temperatur von wenigstens 13°C aufweist.

7. Backhefe-Stamm nach Anspruch 5, der aus einer Gruppe ausgewählt ist, die aus den Stämmen Saccharomyces cerevisiae NCIMB 40328, 40329 und 40330 besteht.

8. Backhefe-Stamm nach Anspruch 6, der aus einer Gruppe ausgewählt ist, die aus den Stämmen Saccharomyces cerevisiae NCIMB 40331 und 40332 besteht.

9. Verwendung eines Backhefe-Stamms nach einem der Ansprüche 5 bis 8 bei der Herstellung von Backwaren zum Backen im Ofen nach einer Konservierung unter Kühlung.

10. Verwendung eines Backhefe-Stamms nach einem der Ansprüche 5 bis 8 als Indikator des Überschreitens der Temperatur in Lebensmittelprodukten für eine Konservierung unter Kühlung.

## Claims

1. A process for constructing a diploid strain of baking yeast having an lti property, i.e. the ability to produce less than 20 ml of CO₂ per g of pressed yeast after 7 d in a maltose medium refrigerated to 3-10°C and to recover its activity from 13-14°C, in which a haploid strain of Saccharomyces cerevisiae is subjected to a mutagenic treatment, at least one mutant having an lti property is selected and is backcrossed at least once with a wild haploid strain of Saccharomyces cerevisiae having an opposite mating type, at least two backcross segregants having an lti property and opposite mating types are selected and are crossed at least once and a diploid strain thus obtained having a growth potential, an lti property and an ability to raise a dough is selected.

2. A process as claimed in claim 1, characterized in that, to verify the lti property of the said diploid strain to be selected, it is subjected to a CO₂ production test in a nutrient medium containing maltose as carbon source for several days at temperatures in the range from 3 to 14°C.

3. A process for constructing a polyploid strain of baking yeast having an lti property, i.e. the ability to produce less than 30 ml of CO₂ per g of pressed yeast after 7 d in a maltose medium refrigerated to 3-9°C and to recover its activity from 13-14°C, in which a polyploid strain of Saccharomyces cerevisiae is optionally subjected to a mutagenic treatment and is then sporulated, at least one segregant having an lti property is selected and is backcrossed at least once with another segregant of this strain having an opposite mating type, at least two backcross segregants having an lti property and opposite mating types are selected and are crossed at least once and a polyploid strain thus obtained having a growth potential, an lti property and an ability to raise a dough is selected.

4. A process as claimed in claim 3, characterized in that, to verify the lti property of the said polyploid strain to be selected, it is subjected to a CO₂ production test in a nutrient medium containing maltose as carbon source for several days at temperatures in the range from 3 to 14°C.

5. A diploid strain of the baking yeast Saccharomyces cerevisiae which has a growth potential in the fed batch process, an ability to raise a dough, a CO₂ production level of less than 20 ml per g pressed yeast after 7 d in maltose medium refrigerated to 3-10°C and a CO₂ production level of at least 40 ml per g pressed yeast after 6 d in maltose medium kept at a temperature of at least 14°C.

6. A polyploid strain of the baking yeast Saccharomyces cerevisiae which has a growth potential in the fed batch process, an ability to raise a dough, a CO₂ production level of less than 30 ml per g pressed yeast after 7 d in maltose medium refrigerated to 3-9°C and a CO₂ production level of at least 60 ml per g pressed yeast after 6 d in maltose medium kept at a temperature of at least 13°C.

7. A baking yeast strain according to claim 5 selected from the group comprising the Saccharomyces cerevisiae strains NCIMB 40328, 40329 and 40330.

8. A baking yeast strain according to claim 6 selected from the group comprising the Saccharomyces cerevisiae strains NCIMB 40331 and 40332.

9. The use of a strain of baking yeast according to claims 5 to 8 in the production of bakery articles to be cooked in an oven after refrigerated storage.

10. The use of a strain of baking yeast according to any of claims 5 to 8 as an over-temperature indicator in food products to be stored under refrigeration.
